# EUROPEAN PATENT APPLICATION

(11) **EP 3 357 401 A1**
(43) Date of publication of application: **08.08.2018**
(21) Application number: 16850814.1
(22) Date of filing: 13.07.2016
(51) Int. Cl.: A61B 1/00

(54) **OCT CATHETER**

(30) Priority: 02.10.2015 JP 2015196521
(71) Applicant: Sumitomo Electric Industries, Ltd., Osaka-shi, Osaka 541-0041 (JP)
(72) Inventor: SASAKI, Dai, Yokohama-shi Kanagawa 244-8588 (JP); TAMEKUNI, Yoshikyo, Yokohama-shi Kanagawa 244-8588 (JP); WATANABE, Takuro, Yokohama-shi Kanagawa 244-8588 (JP)
(74) Representative: Hoffmann Eitle
(86) International application number: PCT/JP2016/070671
(87) International publication number: WO 2017/056637

(57) **Abstract**

To provide an OCT catheter configured such that the movement of an interior member in the front-rear direction in an outer housing is suppressed. An OCT catheter includes an interior member 20 including a torque wire 21 and an optical fiber 25, the torque wire 21 transmitting rotation from a proximal end toward a distal end, the optical fiber 25 being provided in a hollow portion of the torque wire 21 and transmitting light. The OCT catheter further includes an outer housing 30 covering the interior member 20. The outer housing 30 includes a pair of stepped portions (a wall portion 36a and a stepped portion 33a) that face each other in a lengthwise direction of the torque wire 21. The torque wire 21 includes a torque-wire body 22 and a flange portion 23 provided on the torque-wire body 22. The flange portion 23 is provided between the pair of stepped portions. A coil spring 48 is provided between one of the pair of stepped portions and the flange portion 23. The flange portion 23 is pressed toward the other of the pair of stepped portions by the coil spring 48.

## Description

### Technical Field

This application claims the benefit of Japanese Patent Application No. 2015-196521 filed October 2, 2015.

The present invention relates an OCT catheter.

### Background Art

Optical coherence tomography (OCT) catheters for acquiring in vivo optical coherence tomographic images are known. For example, a catheter disclosed by Japanese Unexamined Patent Application Publication No. 2013-202295 includes an optical connector connected to an optical fiber at the proximal end, a condensation-deflection optical system optically connected to the optical fiber at the distal end, and a support tube and a jacket tube both enveloping the optical fiber and extending along the optical fiber.

In general, an OCT catheter that is to be inserted into a blood vessel is used for acquiring an optical coherence tomographic image in a predetermined lengthwise range of an outer housing, such as the jacket tube, while a condensation-deflection optical system is moved backward (pulled backward) relative to the outer housing. Hence, in such an OCT catheter, the condensation-deflection optical system is movable in the lengthwise direction of the outer housing. However, to acquire an optical coherence tomographic image at a fixed position in a situation such as a fundus examination, the condensation-deflection optical system needs to be set stationary at that position.

Moreover, there is typically a clearance between an interior member, such as the condensation-deflection optical system, and the outer housing so that the rotation of the interior member is not hindered by the outer housing. Therefore, depending on the state of use, the interior member may move in the front-rear direction in the outer housing.

### Summary of Invention

### Technical Problem

The present invention provides an OCT catheter configured such that the movement of an interior member in the front-rear direction in an outer housing is suppressed.

### Solution to Problem

An OCT catheter according to the present invention includes an interior member including a tubular torque wire and an optical fiber, the torque wire including a torque-wire body and a flange provided on the torque-wire body, the torque wire transmitting rotation from a proximal end toward a distal end, the optical fiber being enveloped by the torque wire and transmitting light; an outer housing including a first stepped portion and a second stepped portion that face each other across the flange, the outer housing covering the interior member; and an elastic member provided between the first stepped portion and the flange portion and pressing the flange toward the second stepped portion.

A friction-reducing portion may be provided between the elastic member and the flange portion. Likewise, a friction-reducing portion may be provided between the second stepped portion and the flange portion. The friction-reducing portion may be a bearing. The second stepped portion may be positioned nearer to the distal end than the first stepped portion in a lengthwise direction. The flange portion may be a tubular member fixed to the torque-wire body or a thick portion of the torque wire.

### Advantageous Effects of Invention

In the OCT catheter according to the present invention, the movement of the interior member in the front-rear direction in the outer housing can be suppressed.

### Brief Description of Drawings

Figure 1 is a conceptual diagram illustrating an OCT apparatus including an OCT catheter.
Figure 2 is a sectional view of an OCT catheter according to a first embodiment of the present invention.
Figure 3 is a sectional view of an OCT catheter according to a second embodiment of the present invention.
Figure 4 is a sectional view of an OCT catheter according to a third embodiment of the present invention.

### Description of Embodiments

Embodiments of the present invention will now be described specifically with reference to the drawings. As a matter of convenience, elements that are substantially identical are denoted by the same reference numerals, and description of such elements may be omitted. In the following description, the lengthwise direction of the OCT catheter is also referred to as the front-rear direction, with the proximal side defined as the rear side and the distal side defined as the front side.

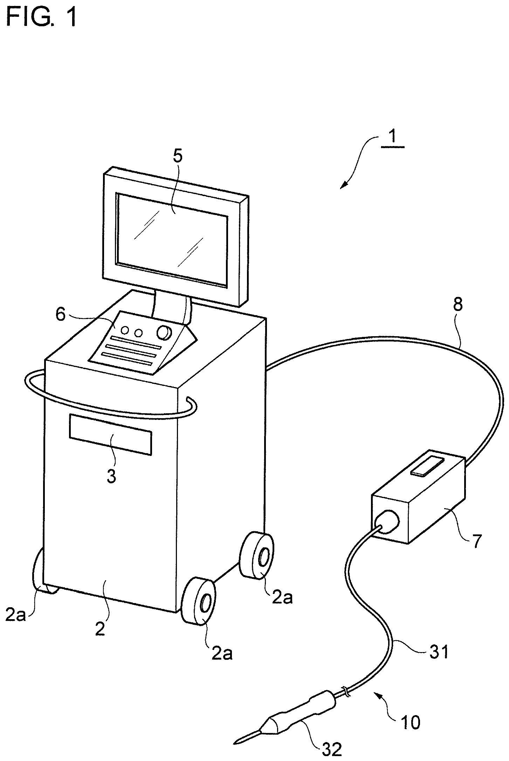

Figure 1 is a conceptual diagram illustrating a medical imaging system 1 for acquiring an optical coherence tomographic image. The medical imaging system 1 includes a console body 2, a driving device 7, and an OCT catheter 10.

The console body 2 includes elements that realize basic functions for acquiring in vivo images, such as a light source, an optical measurement unit, a control unit, a computer that performs image calculation, and a power source. The console body 2 is provided with casters 2a so that the operator can move the console body 2 easily. The console body 2 is also provided in an upper part thereof with a drive 3 into which a recording medium is to be inserted, a monitor 5 that displays an image acquired, and input means 6, such as a keyboard or a mouse, for enabling input operation.

The driving device 7 is a device that rotates an interior member 20 (to be described below) for a rotational scanning operation performed by the OCT catheter 10. The driving device 7 is connected to the console body 2 with a cable 8. The cable 8 includes an electric wire and an optical fiber. The driving device 7 is positioned away from the console body 2 but near the patient. The OCT catheter 10 connected to the driving device 7 includes a handpiece 32. The handpiece 32 is connected to the driving device 7 with a tube 31.

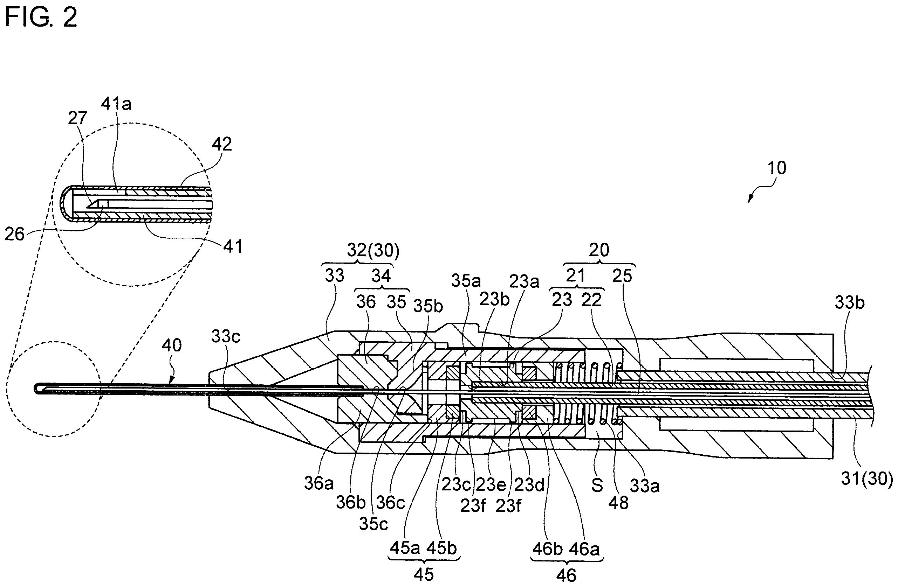

Figure 2 is a sectional view of the OCT catheter 10, which is based on a first embodiment of the present invention. As illustrated in Figure 2, the OCT catheter 10 includes the interior member 20 including a torque wire 21 and an optical fiber 25, and an outer housing 30 covering the interior member 20. The optical fiber 25 is a typical single-mode optical fiber and has a structure in which grass fibers including a core having a high refractive index and a cladding having a low refractive index are covered with a resin covering. The proximal end of the optical fiber 25 is fixed to an optical connector provided in the driving device 7, whereby the optical fiber 25 is optically connected to the console body 2 with the cable 8. The optical fiber 25 is also fixed to the torque wire 21 with adhesive or the like, thereby being rotatable together with the torque wire 21.

At the distal end of the optical fiber 25, provided are a graded-index (GRIN) lens serving as a condensation optical system 26 and a mirror serving as a deflection optical system 27 that are fusion-spliced to each other in series. The condensation optical system 26 condenses light emitted from the distal end of the optical fiber 25. The deflection optical system 27 deflects the light emitted from the distal end of the optical fiber 25 such that the light is redirected substantially perpendicularly to the lengthwise direction of the OCT catheter. The lens (the condensation optical system) and the mirror (the deflection optical system) are each made of silica glass or borosilicate glass.

The torque wire 21 includes a torque-wire body 22 and a flange portion 23 provided on the torque-wire body 22. The torque-wire body 22 is a metal tubular flexible member and has an inside diameter and an outside diameter that are each uniform in the lengthwise direction. The torque-wire body 22 envelops in a hollow portion thereof the optical fiber 25, which transmits light. The torque-wire body 22 transmits, to the distal end of the OCT catheter 10, a rotational force transmitted from the driving device 7 connected to the proximal end thereof.

The flange portion 23 has a larger outside diameter than the torque-wire body 22. The flange portion 23 according to the first embodiment is a tubular member that is fixed to the torque-wire body 22. The flange portion 23 has a through hole 23a through which the torque-wire body 22 extends. At the distal end of the through hole 23a, a stepped portion 23b that is in contact with the distal end of the torque-wire body 22 is provided. The torque-wire body 22 and the flange portion 23 are bonded and fixed to each other with the torque-wire body 22 being in contact with the stepped portion 23b. The flange portion 23 according to the first embodiment is a member that is separate from the torque-wire body 22. Therefore, the flange portion 23 can be easily formed into a desired shape. Moreover, the flange portion 23 may be made of any of various materials such as a low-friction material.

The flange portion 23 includes a distal-end part 23c at the distal end thereof in the axial direction, and a rear-end part 23d at the rear end thereof in the axial direction. The flange portion 23 further includes a middle part 23e provided between the distal-end part 23c and the rear-end part 23d. The end faces of the distal-end part 23c and the rear-end part 23d are each a flat surface perpendicular to the axial direction. The outside diameters of the distal-end part 23c and the rear-end part 23d are smaller than the outside diameter of the middle part 23e. Projections 23f are provided between the distal-end part 23c and the middle part 23e and between the rear-end part 23d and the middle part 23e, respectively. The projections 23f each have an annular shape with an outside diameter larger than the outside diameter of the middle part 23e.

The outer housing 30 includes the tube 31 and the handpiece 32. The tube 31 is a tubular flexible member and envelops the torque-wire body 22. A predetermined clearance is provided between the inner periphery of the tube 31 and the outer periphery of the torque-wire body 22. Hence, the torque-wire body 22 is rotatable in the tube 31. The tube 31 is connected to the driving device 7 at the proximal end thereof and to the handpiece 32 at the distal end thereof.

The handpiece 32 is a portion of the OCT catheter 10 that is to be held by the user. The handpiece 32 includes a substantially cylindrical case body 33 that has an internal space S, and a positioning member 34 that is fixed in the internal space S and determines the position of the interior member 20 in the radial direction. An annular stepped portion (first stepped portion) 33a is provided in the internal space S of the case body 33. The stepped portion 33a projects from the inner peripheral wall of the case body 33 toward the center in the radial direction. The case body has at the rear end thereof a through hole 33b, in which a distal portion of the tube 31 is fitted. The distal end of the tube 31 extends up to the stepped portion 33a and is bonded to, for example, the inner peripheral surface of the stepped portion 33a. A distal portion of the torque wire 21 extends beyond the distal end of the tube 31.

The positioning member 34 includes a first member 35 having a substantially cylindrical shape, and a second member 36 fitted to the distal end of the first member 35. Specifically, the positioning member 34 includes a cylindrical portion 35a included in the first member 35, and a wall portion (second stepped portion) 36a included in the second member 36. The wall portion 36a and the stepped portion 33a face each other in the lengthwise direction of the interior member 20 in the internal space S of the handpiece 32. The wall portion 36a is provided on a side of the cylindrical portion 35a that is nearer to the distal end of the cylindrical portion 35a, and extends radially inward from the inner peripheral surface of the cylindrical portion 35a. The wall portion 36a has in the radial center thereof a through hole 36b through which the optical fiber 25 extends. The rear end of the wall portion 36a forms an end face 36c that is exposed in the cylindrical portion 35a. In the first embodiment, the second member 36 is in engagement with a projected portion 35b included in the first member 35 and extending toward the radial center. The projected portion 35b has a through hole 35c communicating with the through hole 36b.

A needle 40 is fixed at the distal end of the through hole 36b provided in the second member 36. The needle 40 is exposed to the outside from a through hole 33c provided at the distal end of the case body 33. The needle 40 includes a tubular needle body 41 made of, for example, stainless steel, and a resin covering member 42 covering the needle body 41. The needle body 41 envelops a distal portion of the optical fiber 25 such that the distal portion is rotatable therein. The needle body 41 has a slit 41a at the distal end thereof. Hence, the light from the optical fiber 25 is allowed to emerge to the outside.

The flange portion 23 of the torque wire 21 is positioned on the inner side of the cylindrical portion 35a and between the wall portion 36a and the stepped portion 33a. The inside diameter of the cylindrical portion 35a and the outside diameter of the projections 23fof the flange portion 23 are substantially the same. Hence, the flange portion 23 is rotatable on the inner side of the cylindrical portion 35a with the radial position thereof being fixed.

A bearing (friction-reducing portion) 45 is provided on the inner side of the cylindrical portion 35a and between the wall portion 36a and the flange portion 23. The bearing 45 includes an outer race 45a and an inner race 45b provided on the inner side of the outer race 45a. Rotating bodies such as balls, which are not illustrated, are provided between the inner race 45b and the outer race 45a, whereby the inner race 45b is rotatable relative to the outer race 45a. According to the first embodiment, only the outer race 45a faces toward the wall portion 36a (the end face 36c), whereas the inner race 45b faces toward the flange portion 23. The outside diameter of the inner race 45b is larger than the outside diameter of the distal-end part 23c of the flange portion 23. Hence, the distal-end part 23c is in contact with only the inner race 45b and is out of contact with the outer race 45a. The inside diameter of the cylindrical portion 35a and the outside diameter of the bearing 45 are substantially the same, whereby the bearing 45 is movable in the front-rear direction on the inner side of the cylindrical portion 35a. The optical fiber 25 extends on the inner side of the inner race 45b of the bearing 45.

Another bearing (friction-reducing portion) 46 is provided on the inner side of the cylindrical portion 35a and between the stepped portion 33a and the flange portion 23. The bearing 46 has the same configuration as the bearing 45 and includes an outer race 46a and an inner race 46b. Only the outer race 46a faces toward the stepped portion 33a, whereas the inner race 46b faces toward the flange portion 23. The outside diameter of the inner race 46b is larger than the outside diameter of the rear-end part 23d of the flange portion 23. Therefore, the rear-end part 23d of the flange portion 23 is in contact with only the inner race 46b and is out of contact with the outer race 46a. An elastic member is provided between the bearing 46 and the stepped portion 33a (that is, the bearing 46 and the elastic member are provided between the stepped portion 33a and the flange portion 23). In the first embodiment, a coil spring 48 is employed as the elastic member.

The coil spring 48 has a natural length that is longer than a length obtained by subtracting the total length of the bearing 45, the flange portion 23, and the bearing 46 in the front-rear direction from the distance between the wall portion 36a (specifically, the end face 36c) and the stepped portion 33a. Therefore, in a state where the bearing 45, the flange portion 23, the bearing 46, and the coil spring 48 are placed on the inner side of the cylindrical portion 35a, the coil spring 48 presses the bearing 45, the flange portion 23, and the bearing 46 toward the wall portion 36a.

In the OCT catheter 10 described above, the flange portion 23 is pressed in a direction from the stepped portion 33a toward the wall portion 36a by the coil spring 48. Thus, the position of the flange portion 23 in the lengthwise direction of the torque wire 21 is controlled. That is, if the flange portion 23 receives an external force acting in a direction toward the stepped portion 33a, the movement of the flange portion 23 is regulated by the elastic force (urging force) exerted by the coil spring 48. On the other hand, if the flange portion receives external force acting in a direction toward the wall portion 36a, the movement of the flange portion 23 is regulated by the wall portion 36a. The flange portion 23 is provided on the torque wire 21. Therefore, regulating the movement of the flange portion 23 regulates the movements of the torque wire 21 and the optical fiber 25.

During a measurement performed by the medical imaging system 1, the interior member 20 is rotated at a high speed by the driving device 7. If the interior member 20 is moved in the front-rear direction in the outer housing with vibrations or the like caused by such a rotation, the position of the optical fiber becomes instable, making it difficult to acquire an accurate optical coherence tomographic image. According to the first embodiment, the movement of the interior member 20 in the front-rear direction in the outer housing 30 can be suppressed. Therefore, the position of the optical fiber 25 in the needle 40 is kept stable.

The tube 31 and the torque-wire body 22 are arranged with a predetermined clearance interposed therebetween so that the rotation of the interior member 20 is not hindered. Therefore, depending on the state of the tube 31, for example, in a case where the tube 31 is rolled up with a small diameter, the torque-wire body 22 may be pulled in the tube 31 toward the driving device 7, and an excessive load may be applied to the interior member 20. In contrast, according to the first embodiment, the flange portion 23 is pressed toward the distal side by the coil spring 48. Therefore, even if the interior member 20 is pulled toward the proximal side, the coil spring 48 is compressed and thus prevents the interior member 20 from receiving an excessive load. Furthermore, when the interior member 20 that has been pulled is released, the interior member 20 is caused to return to the initial position by the coil spring 48.

Furthermore, since the bearings 45 and 46 are provided as the friction-reducing portions, the rotation of the flange portion 23 on the torque wire 21 is not hindered. Moreover, since the bearings 45 and 46 are employed as the friction-reducing portions, friction can be reduced efficiently.

### Second Embodiment

An OCT catheter 110 according to a second embodiment differs from the OCT catheter 10 according to the first embodiment in the position of the coil spring 48 serving as the elastic member. Now, such differences from the first embodiment will be described mainly. Elements and members that are identical to those described above are denoted by corresponding ones of the reference numerals, and detailed description of such elements and members is omitted.

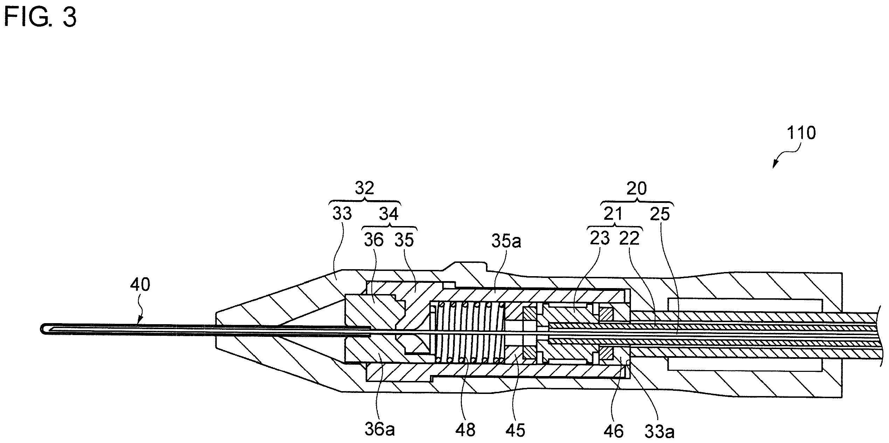

As illustrated in Figure 3, the OCT catheter 110 includes the interior member 20 including the optical fiber 25, the torque-wire body 22, and the flange portion 23; the outer housing 30 covering the interior member 20; and the needle 40. The OCT catheter 110 further includes the bearings 45 and 46 and the coil spring 48 that are provided on the inner side of the cylindrical portion 35a of the first member 35 included in the outer housing 30.

According to the second embodiment, the coil spring 48 is provided between the wall portion (first stepped portion) 36a and the flange portion 23, and the bearing 45 is provided between the coil spring 48 and the flange portion 23. Furthermore, the bearing 46 is provided between the stepped portion (second stepped portion) 33a and the flange portion 23. Therefore, in a state where the coil spring 48, the bearing 45, the flange portion 23, and the bearing 46 are placed on the inner side of the cylindrical portion 35a, the coil spring 48 presses the bearing 45, the flange portion 23, and the bearing 46 toward the stepped portion 33a. Hence, the position of the interior member 20 in the front-rear direction in the outer housing 30 is regulated. Consequently, the position of the optical fiber 25 in the needle 40 is kept stable.

### Third Embodiment

An OCT catheter 210 according to a third embodiment differs from the OCT catheter 10 according to the first embodiment in the configuration of a flange portion 223 provided on the torque-wire body 22. Now, such differences from the first embodiment will be described mainly. Elements and members that are identical to those described above are denoted by corresponding ones of the reference numerals, and detailed description of such elements and members is omitted.

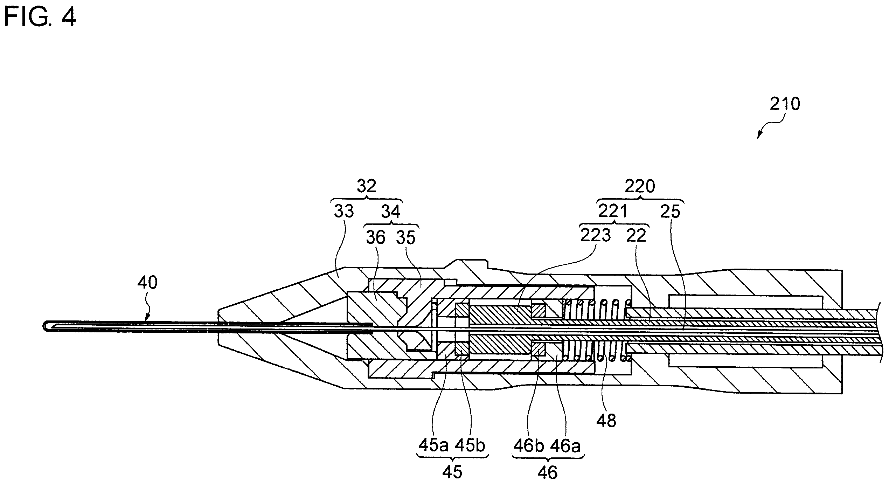

As illustrated in Figure 4, the OCT catheter 210 includes an interior member 220 including the optical fiber 25 and a torque wire 221; the outer housing 30 covering the interior member 220; and the needle 40. The OCT catheter 210 further includes the bearings 45 and 46 and the coil spring 48 that are provided on the inner side of the cylindrical portion 35a of the first member 35 included in the outer housing 30. The arrangement of the bearings 45 and 46 and the coil spring 48 is the same as in the first embodiment.

The torque wire 221 includes the torque-wire body 22 and the flange portion 223. According to the third embodiment, the flange portion 223 forms a thick portion of the torque wire 221 and is provided at the distal end of the torque-wire body 22. The outside diameter of the flange portion 223 is smaller than the outside diameters of the inner races 45b and 46b of the respective bearings 45 and 46. Therefore, the front end and the rear end of the flange portion 223 are out of contact with the outer races 45a and 46a but are in contact with the inner races 45b and 46b. Therefore, as in the first embodiment, the position of the interior member 220 in the front-rear direction in the outer housing 30 is regulated. Consequently, the position of the optical fiber 25 in the needle 40 is kept stable. Since the thick portion of the torque wire 221 serves as the flange portion 223, the number of components can be reduced, which is advantageous in terms of cost and reliability.

While some embodiments of the present invention have been described above with reference to the drawings, the specific configuration is not limited to such embodiments. For example, each friction-reducing portion only needs to be capable of reducing the friction occurring between the flange portion and each of the wall portion 36a, the stepped portion 33a, and the coil spring 48 and may be, for example, a so-called unlubricated bush or the like. Furthermore, the elastic member only needs to be configured to press the flange portion toward the wall portion 36a or toward the stepped portion 33a and may be, for example, a rubber member or the like having a predetermined elasticity. Moreover, some of the features of different embodiments may be combined together. For example, the flange portion 23 according to the second embodiment may be replaced with the flange portion 223 according to the third embodiment.

## Claims

1. An OCT catheter comprising:
an interior member including a tubular torque wire and an optical fiber, the torque wire including a torque-wire body and a flange provided on the torque-wire body, the torque wire transmitting rotation from a proximal end toward a distal end, the optical fiber being enveloped by the torque wire and transmitting light;
an outer housing including a first stepped portion and a second stepped portion that face each other across the flange, the outer housing covering the interior member; and
an elastic member provided between the first stepped portion and the flange and pressing the flange toward the second stepped portion.

2. The OCT catheter according to Claim 1,
wherein a friction-reducing portion is provided between the elastic member and the flange.

3. The OCT catheter according to Claim 1 or 2,
wherein a friction-reducing portion is provided between the second stepped portion and the flange.

4. The OCT catheter according to Claim 2 or 3,
wherein the friction-reducing portion is a bearing.

5. The OCT catheter according to any of Claims 1 to 4,
wherein the second stepped portion is positioned nearer to the distal end than the first stepped portion.

6. The OCT catheter according to any of Claims 1 to 5,
wherein the flange is a tubular member fixed to the torque-wire body.

7. The OCT catheter according to any of Claims 1 to 5,
wherein the flange is a thick portion of the torque wire.
